# EUROPÄISCHE PATENTSCHRIFT

(11) **EP 0 953 559 B1**
(45) Veröffentlichungstag und Bekanntmachung des Hinweises auf die Patenterteilung: **26.03.2003**
(21) Anmeldenummer: 99107938.5
(22) Anmeldetag: 22.04.1999
(51) Int. Cl.: C07C 45/34, C07C 47/06, B01D 45/00

(54) **Verbesserte Trennung des Katalysators bei einem Verfahren zur Oxidation eines Reaktionsgases**
Improved separation of the catalyst in a process for the oxydation of a gas
Procédé amélioré de séparation du catalyseur dans une réaction d'oxydation d'un gas

(30) Priorität: 30.04.1998 DE 19819317
(43) Veröffentlichungstag der Anmeldung: 03.11.1999
(73) Patentinhaber: Celanese Chemicals Europe GmbH, 60439 Frankfurt am Main (DE)
(72) Erfinder: Rinne, Bernd, 65929 Frankfurt (DE); Franken-Stellamans, Erhard Dr., 65527 Niedernhausen (DE)

(56) Entgegenhaltungen:
- EP-A- 0 006 523
- EP-A- 0 438 251
- WO-A-97/47372
- DE-A- 2 521 092
- DE-B- 1 190 451

## Beschreibung

Die Erfindung betrifft ein Verfahren zur Oxidation eines Reaktionsgases in Gegenwart einer Katalysatorlösung, bei dem man
a) das Reaktionsgas in einem Reaktor in Gegenwart der Katalysatorlösung oxidiert, wobei ein Reaktionsgemisch entsteht, das Tröpfchen der Katalysatorlösung enthält, und das man
b) in einen ersten Abscheider leitet und in diesem Tröpfchen der Katalysatorlösung aus dem Reaktionsgemisch entfernt und bei dem man
c) das verbleibende Reaktionsgemisch aus dem ersten Abscheider in einen Waschapparat leitet und in diesem das Oxidationsprodukt aus dem Reaktionsgemisch entfernt.

Das genannte Verfahren ist aus dem Stand der Technik bekannt (DE 1 190 451) und wird zur großindustriellen Herstellung von Aldehyden, Ketonen oder den Aldehyden entsprechenden Säuren durch Oxidation von Olefinen verwendet. Insbesondere wird nach diesem Verfahren Acetaldehyd aus Ethylen und Sauerstoff hergestellt. Zur Katalyse der stark exothermen Oxidationsreaktion mit Sauerstoff als Oxidationsmittel wird üblicherweise eine wäßrige Lösung von Kupferchlorid und Palladiumchlorid verwendet.

Die Oxidation findet in einem Reaktor bei der Siedetemperatur der wäßrigen Katalysatorlösung statt. Die Reaktionswärme wird in erster Linie durch Verdampfung von Wasser und Acetaldehyd aus der Katalysatorlösung abgeführt (Siedekühlung). Das entstehende Reaktionsgemisch, enthaltend im wesentlichen Flüssig-Katalysator, Wasserdampf, gasförmigen Acetaldehyd und Nebel aus Katalysatorlösung, wird in einen ersten Abscheider, einen sogenannten Nebelfänger geleitet, in dem die Dampf-/Gas-Nebelanteile größtenteils aus dem Reaktionsgemisch entfernt werden, beispielsweise mittels Waschwasser, das im Gegenstrom in den Nebelfänger eingedüst wird. Das verbleibende Reaktionsgemisch wird anschließend in Wärmetauschern abgekühlt, wobei katalysatorhaltige Kondensatströme mit teils bereits nennenswertem Acetaldehyd-Gehalt anfallen, und in einem Waschapparat, üblicherweise eine Waschkolonne, mit Wasser ausgewaschen, wobei sich die Hauptmenge des noch in der Gasphase befindlichen Acetaldehyds im Wasser löst. Die Waschphase, vereint mit den durch eine erhöhte Acetaldehyd-Konzentration gekennzeichneten Kondensatströmen, wird zur Aufarbeitung abgezogen. Das nun verbleibende Reaktionsgemisch, das noch geringe Mengen Acetaldehyd enthält, wird als Kreisgas in den Reaktor zurückgeführt. Katalysatorhaltiges Kondensat mit nur geringem Acetaldehyd-Gehalt wird als Waschwasser für den Nebelfänger verwendet.

Nachteilig an dem bekannten Verfahren ist, daß die Abtrennung des Katalysators nicht vollständig ist. Dies führt erstens zu Verlusten an wertvollem Edelmetallkatalysator und Kupfer, was auch die Oxidationsreaktion nachteilig beeinflußt, und zweitens zu erhöhten Entsorgungskosten, da der Katalysator und das Kupfer in das Abwasser der Aufarbeitungsanlage gelangen und mit diesem umweltgerecht entsorgt werden müssen.

Der Erfindung lag daher die Aufgabe zugrunde, die Abtrennung des Katalysators zu verbessern.

Diese Aufgabe wird erfindungsgemäß dadurch gelöst, daß man das Reaktionsgemisch aus dem ersten Abscheider durch einen zweiten Abscheider leitet, der dem ersten Abscheider nachgeschaltet und dem Waschapparat vorgeschaltet ist, und in dem zweiten Abscheider restliche Katalysatorlösung aus dem Reaktionsgemisch entfernt.

Gegenstand der Erfindung ist somit ein Verfahren zur Oxidation von Olefinen zu Aldehyden oder Ketonen oder von Aldehyden zu den entsprechenden Carbonsäuren in Gegenwart einer wäßrigen Katalysatorlösung, bei dem man
a) das Reaktionsgas in einem Reaktor in Gegenwart der Katalysatorlösung oxidiert, wobei ein Reaktionsgemisch entsteht, das Tröpfchen der wäßrigen Katalysatorlösung enthält, und das man
b) in einen ersten Abscheider leitet und in diesem Tröpfchen der wäßrigen Katalysatorlösung aus dem Reaktionsgemisch entfernt und bei dem man
c) das verbleibende Reaktionsgemisch aus dem ersten Abscheider in einen Waschapparat leitet und in diesem das Oxidationsprodukt aus dem Reaktionsgemisch entfernt,
   dadurch gekennzeichnet, daß man
d) das Reaktionsgemisch aus dem ersten Abscheider durch einen Drallabscheider leitet, der Mittel zur Erzeugung einer Drehströmung aufweist und der dem ersten Abscheider nachgeschaltet und dem Waschapparat vorgeschaltet ist, und in dem Drallabscheider restliche wäßrige Katalysatorlösung aus dem Reaktionsgemisch entfernt.

Gegenstand der Erfindung ist insbesondere das entsprechende Verfahren zur Herstellung von Acetaldehyd aus Ethylen und Sauerstoff. Bevorzugte Ausgestaltungen ergeben sich aus den Unteransprüchen. Es können auch einzelne oder mehrere der in den Unteransprüchen offenbarten Ausgestaltungen jeweils für sich oder in Kombination Lösungen der zugrundeliegenden Aufgabe darstellen und es sind auch die einzelnen Merkmale innerhalb der Anspruchskategorien beliebig kombinierbar.

Als zweite Abscheider können mit Vorteil sogenannte Drallabscheider oder Dralltropfenabscheider verwendet werden. In diesen wird der Strom des Reaktionsgemisches durch entsprechende Einbauten wie Umlenkschaufeln in eine spiralförmige Bahn gezwungen, so daß eine Drehströmung entsteht, wodurch aufgrund der auftretenden Zentrifugalkräfte im Gasstrom enthaltene Tröpfchen an der äußeren Innenwand des Abscheiders abgeschieden werden. Die abgeschiedenen Tröpfchen können beispielsweise in einer umlaufenden Rinne aufgefangen, abgezogen und in den ersten Waschapparat als Waschwasser zurückgeführt werden.

Die Lösung der der Erfindung zugrunde liegenden Aufgabe ist insofern überraschend als der Durchschnittsfachmann nicht damit rechnen konnte, daß der Gasstrom aus dem ersten Waschapparat kontinuierlich anfällt, da bei exothermen Reaktionen, die unter Siedebedingungen (Siedekühlung) durchgeführt werden, mit stark schwankenden Gasströmungen zu rechnen ist, was gegen den Einsatz eines Drallabscheiders spricht, insbesondere dann, wenn der reale Druckverlust im Abscheider infolge solcher stark unterschiedlichen Gasströmungen nicht bekannt bzw. zugänglich ist. Entsprechend sind auch keine Anwendungen derartiger Abscheider für Gasströmungen im Sattdampfgebiet, d.h. bei Bedingungen, bei denen Gas und Flüssigkeit im Gleichgewicht vorliegen, bekannt. Zusätzlich ist bei diesen Bedingungen die Tropfengrößenverteilung nicht bekannt und auch nicht zu ermitteln. Die Tropfen hätten daher für eine erfolgreiche Abscheidung auch zu klein sein können.

Des weiteren wurde die Anordnung des Abscheiders entgegen den üblichen, dem Fachmann geläufigen Vorgaben gewählt, d. h. die Durchströmung des senkrecht angeordneten Abscheiders findet von oben nach unten statt, die Strömung weist also eine Hauptkomponente in Richtung der Schwerkraft auf. Weiterhin bestand noch die Gefahr, daß sich die Umlenkschaufeln mit Katalysatorablagerungen belegen würden, was die Beschleunigung der Gasströmung in Umfangsrichtung (Drallerzeugung) beeinträchtigt hätte. Weiter überraschend ist, daß weder eine Wendel zum Sammeln der abgeschiedenen Flüssigkeit noch ein Spülanschluß erforderlich sind.

Gegenstand der Erfindung ist daher ebenso die Verwendung eines Drallabscheiders zur Abscheidung von Tröpfchen einer wäßrigen Katalysatorlösung aus Gasströmen im Sattdampfgebiet bei der Oxidation von Olefinen zu Aldehyden oder Ketonen oder von Aldehyden zu den entsprechenden Carbonsäuren.

Die Vorteile des erfindungsgemäßen Verfahrens sind unter anderem darin zu sehen, daß der Verlust an wertvollem Edelmetallkatalysator und, damit einhergehend, die Belastung des Abwassers der Anlage mit Schwermetallen vermindert wird. Außerdem hat sich gezeigt, daß an den Apparaten und Anlagenteilen, die dem Drallabscheider nachgeschaltet sind, Schäden infolge Loch- und/oder Spannungsrißkorrosion zurückgehen bzw. vermieden werden, da weniger der Lochkorrosion und Spannungsrißkorrosion stark begünstigenden Katalysatorlösung in diese Anlagenteile gelangt. Daher können nun preisgünstigere Werkstoffe als bisher für diese Apparate eingesetzt werden. Weiterhin gehen Foulingerscheinungen (Ablagerungen) in der Waschkolonne zurück. Ein weiterer positiver Effekt ist, daß bei sonst gleichen Gegebenheiten die Produktion an Acetaldehyd gesteigert werden kann, und zwar aus folgenden Gründen: Erhöht man den Kreisgasstrom, was im Sinne einer höheren Produktion günstig ist, so kommt es zu einem verstärkten Schäumen der Katalysatorlösung und damit zu einem deutlich verstärkten Austrag an Katalysatorlösung aus dem Reaktor in die nachfolgenden Apparate, mit den genannten Nachteilen. Mit der erfindungsgemäßen Abscheidung ist dieses Verhalten nicht mehr so nachteilig, da infolge der ausgezeichneten Abscheidung die verstärkt ausgetragene Katalysatorlösung wieder zurückgeführt werden kann.

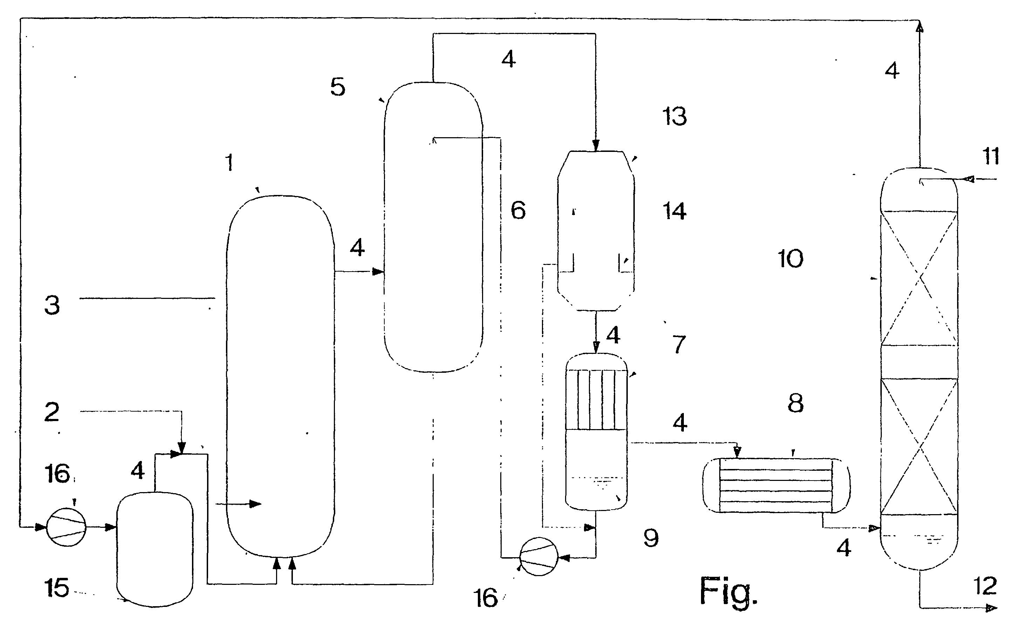

Im folgenden wird eine beispielhafte Ausgestaltung der Erfindung anhand des in der Figur dargestellten Verfahrensfließbildes und anhand von Betriebserfahrungen näher beschrieben. Eine Beschränkung der Erfindung in irgendeiner Weise ist dadurch nicht beabsichtigt.

Der bekannte Teil des Verfahrens wurde weiter oben bereits beschrieben, weshalb hier nur die Bezugsziffern benannt seien. Es bedeutet: 1 Reaktor, 2 Ethylen, 3 Sauerstoff, 4 Reaktionsgemisch, 5 erster Abscheider (Nebelfänger), 6 Waschwasser, 7 ein erster Kühler, 8 ein zweiter Kühler, 9 katalysatorhaltiges Kondensat, 10 Waschapparat (Waschkolonne), 11 Wasser und 12 acetaldehydhaltiges Wasser.

Erfindungsgemäß ist nach dem ersten Waschapparat 5 und vor der Waschkolonne 10, und insbesondere vor dem ersten Kühler 7, ein Drallabscheider 13 als zweiter Abscheider senkrecht angeordnet. In diesem wird das Reaktionsgemisch 4 mittels Umlenkschaufeln (nicht dargestellt) in einer Drehströmung (Pfeil) nach unten geführt. Der Drallabscheider 13 weist innen eine umlaufende Rinne 14 auf. Aus dieser Rinne 14 wird die abgeschiedene Katalysatorlösung abgeführt, mit dem aus dem ersten Kühler 7 gewonnenen katalysatorhaltigen Kondensat 9 vermischt und als Waschwasser 6 in den ersten Waschapparat 5 eingedüst.

### Betriebserfahrungen:

In einer gewerblichen, industriellen Anlage zur Herstellung von Acetaldehyd aus Ethylen und Sauerstoff wurde entsprechend der Fig. erfindungsgemäß ein Dralltropfenabscheider installiert. Ergebnis: Die Anlagenkapazität konnte von 120000 t/a Acetaldehyd auf 135000 t/a erhöht werden, und die Belastung des Abwassers aus der Anlage mit Kupfer und Palladium ging um den Faktor 50 zurück.

## Patentansprüche

1. Verfahren zur Oxidation von Olefinen zu Aldehyden oder Ketonen oder von Aldehyden zu den entsprechenden Carbonsäuren in Gegenwart einer wäßrigen Katalysatorlösung, bei dem man
a) das Reaktionsgas in einem Reaktor in Gegenwart der Katalysatorlösung oxidiert, wobei ein Reaktionsgemisch entsteht, das Tröpfchen der wäßrigen Katalysatorlösung enthält, und das man
b) in einen ersten Abscheider leitet und in diesem Tröpfchen der wäßrigen Katalysatorlösung aus dem Reaktionsgemisch entfernt und bei dem man
c) das verbleibende Reaktionsgemisch aus dem ersten Abscheider in einen Waschapparat leitet und in diesem das Oxidationsprodukt aus dem Reaktionsgemisch entfernt,
**dadurch gekennzeichnet, daß** man
d) das Reaktionsgemisch aus dem ersten Abscheider durch einen Drallabscheider leitet, der Mittel zur Erzeugung einer Drehströmung aufweist und der dem ersten Abscheider nachgeschaltet und dem Waschapparat vorgeschaltet ist, und in dem Drallabscheider restliche wäßrige Katalysatorlösung aus dem Reaktionsgemisch entfernt.

2. Verfahren nach Anspruch 1, **dadurch gekennzeichnet, daß** man zur Erzeugung der Drehströmung Umlenkschaufeln verwendet.

3. Verfahren nach Anspruch 1 oder 2, **dadurch gekennzeichnet, daß** das Reaktionsgas ein Olefin ist, insbesondere Ethylen.

4. Verfahren nach Anspruch 1, **dadurch gekennzeichnet, daß** der Drallabscheider aus Titan gefertigt ist.

5. Verwendung eines Drallabscheiders zur Abscheidung von Tröpfchen einer wäßrigen Katalysatorlösung aus Gasströmen im Sattdampfgebiet bei der Oxidation von Olefinen zu Aldehyden oder Ketonen oder von Aldehyden zu den entsprechenden Carbonsäuren.

## Revendications

1. Procédé d'oxydation d'oléfines en aldéhydes ou en cétones ou d'aldéhydes en acides carboxyliques correspondants en présence d'une solution aqueuse de catalyseur, dans lequel
a) on oxyde le gaz de réaction dans un réacteur en présence de la solution de catalyseur, ce qui entraîne la formation d'un mélange réactionnel contenant des gouttelettes de la solution aqueuse de catalyseur,
b) on fait passer ce mélange réactionnel dans un premier séparateur dans lequel on sépare les gouttelettes de solution aqueuse de catalyseur du mélange réactionnel et
c) on fait passer le mélange réactionnel restant du premier séparateur dans un appareil de lavage dans lequel on sépare le produit d'oxydation du mélange réactionnel,
**caractérisé en ce que**
d) on fait passer le mélange réactionnel issu du premier séparateur à travers un séparateur giratoire, qui présente des moyens de production d'un écoulement rotatif et qui est disposé après le premier séparateur et avant l'appareil de lavage, et on sépare la solution aqueuse de catalyseur restante du mélange réactionnel dans le séparateur giratoire.

2. Procédé selon la revendication 1, **caractérisé en ce que** l'on utilise des déflecteurs en formes de pales pour produire l'écoulement rotatif.

3. Procédé selon la revendication 1 ou 2, **caractérisé en ce que** le gaz de réaction est une oléfine, en particulier l'éthylène.

4. Procédé selon la revendication 1, **caractérisé en ce que** le séparateur giratoire est construit en titane.

5. Utilisation d'un séparateur giratoire pour la séparation de gouttelettes d'une solution aqueuse de catalyseur à partir de courants gazeux dans le domaine de la vapeur saturée lors de l'oxydation d'oléfines en aldéhydes ou en cétones ou d'aldéhydes en acides carboxyliques correspondants.

## Claims

1. A process for oxidizing olefins to aldehydes or ketones, or aldehydes to the corresponding carboxylic acids in the presence of an aqueous catalyst solution, in which
a) the reaction gas is oxidized in a reactor in the presence of the catalyst solution, a reaction mixture being formed which comprises droplets of the aqueous catalyst solution, and which
b) is passed into a first separator and in this droplets of the aqueous catalyst solution are removed from the reaction mixture, and in which process
c) the remaining reaction mixture is passed from the first separator into a scrubbing apparatus and in this the oxidation product is removed from the reaction mixture,
which comprises
d) passing the reaction mixture from the first separator through a centrifugal separator having means for generating a rotary flow and which is downstream of the first separator and upstream of the scrubbing apparaturs and, in the centrifugal separator, removing residual aqueous catalyst solution from the reaction mixture.

2. The process as claimed in claim 1, wherein, to generate the rotary flow, deflection blades are used.

3. The process as claimed in claim 1 or 2, wherein the reaction gas is an olefin, in particular ethylene.

4. The process as claimed in claim 1, wherein the centrifugal separator is fabricated from titanium.

5. The use of a centrifugal separator for separating off droplets of an aqueous catalyst solution from gas streams in the saturated vapor region from the oxidation of olefins to aldehydes or ketones, or of aldehydes to the corresponding carboxylic acids.
